# EUROPEAN PATENT APPLICATION

(11) **EP 3 085 339 A1**
(43) Date of publication of application: **26.10.2016**
(21) Application number: 14872567.4
(22) Date of filing: 22.12.2014
(51) Int. Cl.: A61F 2/90

(54) **VASCULAR STENT AND METHOD FOR MANUFACTURING SAME**

(30) Priority: 20.12.2013 CN 201310712278
(71) Applicant: Microport Neurotech (Shanghai) Co., Ltd., Shanghai 201318 (CN)
(72) Inventor: ZHANG, Wanling, Shanghai 201318 (CN); XIE, Zhiyong, Shanghai 201318 (CN); LU, Huina, Shanghai 201318 (CN); YU, Hairui, Shanghai 201318 (CN); ZHANG, Lei, Shanghai 201318 (CN); NI, Huali, Shanghai 201318 (CN)
(74) Representative: Patentship Patentanwaltsgesellschaft mbH
(86) International application number: PCT/CN2014/094461
(87) International publication number: WO 2015/090237

(57) **Abstract**

A luminal stent and a method of fabricating such a luminal stent are disclosed. The luminal stent has a tubular mesh structure formed of metal filaments braided with biodegradable filaments. The luminal stent has the following advantages: 1) it has a thinner wall which facilitates the application of the stent in a narrower vascular lumen with a reduced risk of causing vascular restenosis; 2) it causes a mitigated rejection response of the human body to the presence of the deployed stent as a foreign object and is thus more favorable to tissue endothelialization; and 3) it allows a higher mesh-hole density which makes it possible to change the hemodynamic behavior at a lesion site where the stent is deployed in a more desirable way.

## Description

### TECHNICAL FIELD

The present invention relates generally to the field of medical devices and, in particular, to a luminal stent (such as a vascular stent). Specifically, the invention is directed to a luminal stent in one aspect, and to a method of fabricating such a luminal stent in a second aspect.

### BACKGROUND

With the development of endovascular therapy techniques, various types of covered stents have been widely used. A covered stent is a collapsible luminal stent which is composed mainly of a matrix in the form of a metal support frame and a prosthetic vascular cover attached to an inner or outer wall of the metal support frame. In therapeutic use, the covered stent acts as a conduit which isolates an enlarging vascular lesion or a blood flow to prevent rupture of the lesion due to the enlargement while ensuring a smooth flow of blood. Good results have been obtained from the use of such covered stents in the isolation of various aneurysms and aortic dissections and prevention of their rupture due to enlargement.

However, these covered stents are disadvantageous in that the intra-luminal isolation is sometimes inapplicable due to improper lesion shape or location. For example, in case of a major branch vessel present in the vicinity of a proximal end of an aneurysm or of the entry tear of a dissection, the aneurysm will have a neck portion that is too short to provide sufficient space for proximal anchoring of the prosthesis. As another example, when the lesion is so extensive as to affect both the thoracic aorta and the abdominal aorta or when an aortic dissection has involved a major branch vessel such as the brachiocephalic trunk, left common carotid artery, left subclavian artery or celiac trunk, the deployment of the prosthesis will lead to, apart from isolation of the lesion, blocking of the branch vessel and hence serious consequences. This greatly limits the application and development of endovascular techniques.

In recent years, it has been reported to treat vascular enlargements by coaxially and overlappingly deploying multiple metal mesh stents in an ectatic vessel. Instead of isolating a blood flow from a defective blood vessel, this treatment method alters a blood flow disorder by remodeling a normal laminar flow. In the method, a solid thrombus grows between the metal mesh stent and the diseased vessel wall, which results in repair of the vessel due to normal growth of cells and tissue of the vessel wall. This method can also ensure a laminar blood flow for a branch vessel, i.e., an unblocked branch vessel, at the site where the stent is deployed.

However, the use of the multiple mesh stents is still associated with several drawbacks. One of the drawbacks is that the rigid stents are rather bulky even in a contracted configuration and therefore need to be loaded in a sheath having a large diameter, which is not conducive to delivery of the implant. Another drawback associated with the use of the multiple mesh stents is that they will narrow the lumen where they are deployed, which is undesirable for applications with an originally narrow lumen. A third drawback is that once holes in the part of the stents facing an opening of the branch vessel are clogged with the time elapsing, the rigid nature of the metal stents will make it impossible for another surgical intervention to be applied to the opening of the branch vessel. In this case, an open surgery becomes necessary for vascular anastomosis or the like.

In recent years, there has been also developed three-dimensional metal mesh stents which are metal wire braided integral structures also for rectification of a disorderly blood-flow vortex within a diseased lumen.

Chinese Pat. App. No. CN98102782.2 discloses a composite endovascular stent for medical use, structurally characterized in being formed by the braiding of a predetermined number of 316L stainless steel filaments with a diameter of 0.1-0.4 mm and a predetermined number of tantalum filaments with a diameter of 0.1-0.2 mm. The stent has a diameter of 5-50 mm and a length of 40-100 mm, with each single filament having a helix angle of 30°-60°. The number of tantalum filaments is desired to be 1-3. This endovascular stent has high corrosion resistance, high biocompatibility, high traceability, sufficient elastic deformability and good geometric stability.

However, this three-dimensional mesh stent still has several disadvantages, such as for example: 1) the two types of filaments are both metal filaments tending to have relatively great diameters which lead to a limited mesh-hole density for the stent; and 2) despite the high biocompatibility of the two kinds of filaments, they are still foreign to the human body after all and will induce a certain rejection response which is not conducive to rapid tissue endothelialization after the deployment of the stent.

### SUMMARY OF THE INVENTION

It is therefore an objective of the present invention to overcome the above-described shortcomings of the prior-art luminal stents with the following two main aims: 1) large radial strength and a high mesh-hole density; and 2) rapid tissue endothelialization after the deployment.

The above objective is attained by a luminal stent according to the present invention, which has a tubular mesh structure formed of metal filaments braided with biodegradable filaments. The metal filaments may be characterized in being rather rigid to withstand some forces acting on the stent and having shape memory capabilities and biocompatibility. The biodegradable filaments may be characterized in being flexible, completely degradable, able to allow a higher mesh-hole density of the luminal stent, and free of toxic or side effects on the human body. With time elapsing after the deployment, the biodegradable filaments may degrade by itself without generating any product toxic or adverse to the human body.

In view of short-term treatment effectiveness, as the luminal stent is braided from metal filaments and biodegradable filaments, it has a high mesh-hole density, as well as great radial strength due to the use of the metal filaments. Such a stent with great radial strength and a high mesh-hole density is able to reduce the risk of aneurysm rupture during surgery. In terms of long-term treatment effectiveness, because a highly biocompatible polymeric material is used to fabricate the biodegradable filaments of the luminal stent, and since the luminal stent can carry some therapeutic agent, rapid tissue endothelialization after the deployment of the luminal stent is facilitated.

According to the present invention, the luminal stent may usually be a tubular stent, and the metal filaments are flexible filaments of one or more metals selected from a group consisting of shape memory alloys, stainless steels and platinum-iridium alloys. Preferably, the metal filaments are filaments of one or more shape memory alloys selected from a group consisting of nickel-titanium-based shape-memory alloys, copper-based shape-memory alloys and iron-based shape-memory alloys.

According to the present invention, the biodegradable filaments may be filaments of one or more selected from a group consisting of poly(p-dioxanone) (PPDO), polylactic acid (PLA) and poly(glycolide-co-lactide) (PGLA).

According to embodiments of the present invention, the metal filaments are interlaced with the biodegradable. Preferably, a ratio of a number of the metal filaments to a number of the biodegradable filaments is from 1:1 to 1:3. Alternatively, the ratio of the number of the metal filaments to the number of the biodegradable filaments may be differently selected according to practical needs.

According to other embodiments of the present invention, the metal filaments may be twisted together with the biodegradable filaments to form composite strands, wherein the tubular mesh structure is braided from the composite strands. Preferably, the metal filaments are twisted together with the biodegradable filaments in a ratio of the number of the metal filaments to the number of the biodegradable filaments is from 1:1 to 1:3 to form the composite strands.

Preferably, the luminal stent is an uncovered single-layered stent.

Preferably, the luminal stent is an endovascular stent for medical use.

The present invention also provides a method of fabricating a luminal stent, which includes: forming a lattice by a braiding process; and shaping the lattice into a tubular mesh stent. The method is characterized in that the braiding process includes braiding metal filaments together with biodegradable filaments to form the lattice. In the method, a three-dimensional braiding technique may be used.

According to embodiments of the present invention, the braiding process may include interlacing the metal filaments with the biodegradable filaments. Preferably, a ratio of a number of the metal filaments to a number of the biodegradable filaments is from 1:1 to 1:3.

Alternatively, the braiding process may be accomplished by braiding composite strands, i.e., first forming the composite strands by twisting together the aforementioned two kinds of filaments and then braiding the composite strands. Accordingly, according to other embodiments of the present invention, the method may further include twisting the metal filaments together with the biodegradable filaments to form the composite strands prior to the braiding process, wherein the braiding process includes braiding the composite strands to form the lattice. Preferably, the metal filaments are twisted together with the biodegradable filaments in a ratio of a number of the metal filaments to a number of the biodegradable filaments which are twisted together to form the composite strands is from 1:1 to 1:3.

According to the present invention, the ratio of the numbers of the two types of filaments may be selected according to a desired degradation rate, mechanical property, or mesh-hole density of the luminal stent. In case of a high degradation rate being required, the proportion of the biodegradable filaments may be increased. Similarly, the proportion of the metal filaments may be raised if a slow degradation speed is needed. Additionally, when to increase a mechanical property of the luminal stent, such as radial strength or apposition, the proportion of the metal filaments may be increased. Further, a larger proportion of the biodegradable filaments in the luminal stent will lead to an increased mesh-hole density thereof.

In comparison with the prior art luminal stents, first, the luminal stent of the present invention is a tubular mesh stent which is braided from two kinds of filaments and may assume the form of an uncovered single-layered stent. Therefore, the inventive luminal stent has a thinner wall which facilitates the application of the luminal stent in a narrower vascular lumen with a reduced risk of causing vascular restenosis. Second, the luminal stent of the present invention uses both metal filaments and biodegradable polymeric filaments, and hence take advantage of the desirable mechanical properties of the metal filaments as well as the degradability of the polymeric filaments. This allow partial disappearance of the stent at a certain time after the deployment, which leads to a mitigated rejection response of the human body to the presence of the stent as a foreign object and is thus more favorable to tissue endothelialization. In preferred embodiments, the metal filaments are fabricated from a shape memory alloy which imparts superelasticity and shape memory capabilities to the luminal stent. Third, the use of the diametrically smaller biodegradable polymeric filaments allows the braided luminal stent to have denser mesh holes, thus making it possible to change the hemodynamic behavior at a lesion site where the stent is deployed in a more desirable way.

### BRIEF DESCRIPTION OF THE DRAWINGS

The features and advantages of the present invention will become readily apparent from the following detailed description of a few embodiments thereof, which is to be read in connection with the accompanying drawings. It is apparent that the embodiments set forth below are only a part, but not all, of the possible embodiments of the present invention. In light of the teachings of the embodiments disclosed herein, those skilled in the art can make all other possible embodiments without exerting creative efforts. All such embodiments are also embraced in the scope of this invention. In the drawings:
Fig. 1 is a plan view of a luminal stent in accordance with one embodiment of the present invention;
Fig. 2 is a plan view of a luminal stent in accordance with another embodiment of the present invention; and
Fig. 3 is a plan view of a luminal stent in accordance with yet another embodiment of the present invention.

### DETAILED DESCRIPTION

Some embodiments of the inventive luminal stent are described below with reference to the accompanying drawings.

### Embodiment 1

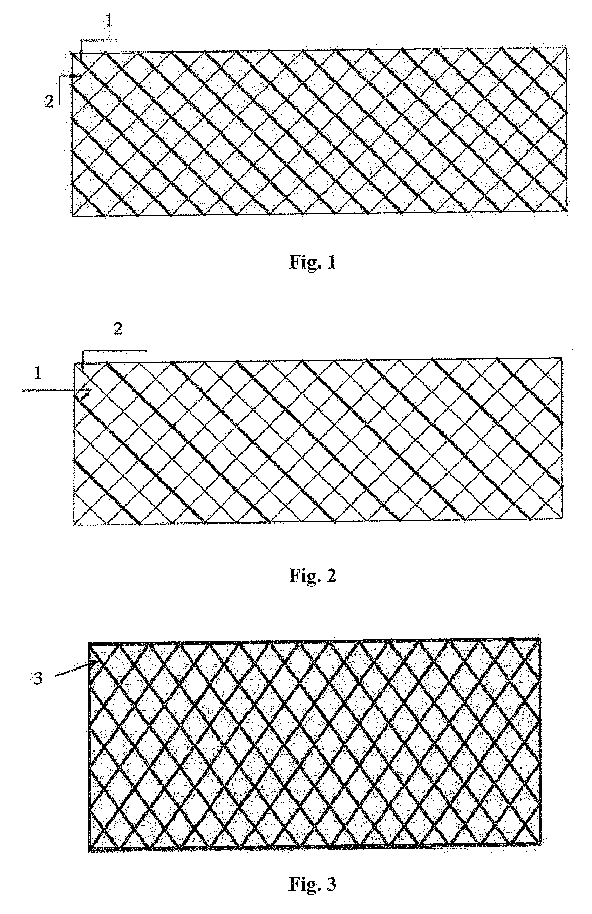

Fig. 1 shows a plan view of a luminal stent constructed in accordance with Embodiment 1 of the present invention. The luminal stent has a tubular mesh structure formed of two materials, i.e., material 1 and material 2. The material 1 is filaments of a shape memory alloy, implemented as a nickel-titanium alloy in this embodiment, having a diameter of 0.0014 inches. The material 2 is filaments of biodegradable fibers, implemented as polylactic acid (PLA) fibers in this embodiment, having a diameter of 0.0012 inches. The two materials are so braided that they are interlaced in a ratio of 1:1 to form a lattice. That is, in the lattice, the numbers of filaments of the two materials are the same. In this embodiment, 24 filaments of each of the materials 1 and 2 are used.

### Embodiment 2

Fig. 2 shows a plan view of a luminal stent constructed in accordance with Embodiment 2 of the present invention. The luminal stent has a tubular mesh structure formed of two materials, i.e., material 1 and material 2. The material 1 is filaments of a shape memory alloy, implemented as a nickel-titanium alloy in this embodiment, having a diameter of 0.0014 inches. The material 2 is filaments of biodegradable fibers, implemented as poly(glycolide-co-lactide) (PGLA) fibers in this embodiment, having a diameter of 0.0012 inches. The two materials are braided such that they are interlaced in a ratio of 1:3 to form a lattice. That is, in the lattice, the number of filaments of the material 2 is three times the number of filaments of the material 1. In this embodiment, totally 48 filaments of the materials 1 and 2 are used.

### Embodiment 3

Fig. 3 shows a plan view of a luminal stent constructed in accordance with Embodiment 3 of the present invention. The luminal stent assumes the form of an uncovered single-layered stent formed of composite strands 3 each consisting of filaments of two materials (A and B) twisted together in a ratio of 1:3. That is to say, one filament of the material A is twisted with 3 filaments of the material B to form one composite strand 3. The material A is flexible filaments, implemented as filaments of a platinum-iridium alloy in this embodiment, having a diameter of 0.0014 inches. The material B is filaments of biodegradable fibers, implemented as PLA fibers in this embodiment, having a diameter of 0.0012 inches.

### Embodiment 4

Referring again to Fig. 3, a luminal stent constructed in accordance with Embodiment 4 of the present invention also assumes the form of an uncovered single-layered stent formed of composite strands. In this embodiment, each strand 3 consists of twisted filaments of three materials (A, B and C). The material A is flexible filaments, implemented as stainless steel filaments in this embodiment, having a diameter of 0.0014 inches. The materials B and C are both filaments of biodegradable fibers. In this embodiment, the material B is implemented as poly(p-dioxanone) (PPDO), and the material C as PLA. Additionally, both of the materials B and C have a filament diameter of 0.0012 inches. Each composite strand 3 is constructed by twisting together filaments of the flexible material (i.e., the material A) and the biodegradable materials (i.e., the materials B and C) in a ratio of 1:2. That is, one flexible filament (A) is twisted together with two biodegradable fiber filaments (one filament of the material B and one filament of the material C) to form a single composite strand 3.

The luminal stents according to the foregoing embodiments of the present invention have at least the following advantages: 1) their single-layered braided form has a thinner wall which allows them to be deployed in a narrow lumen of a blood vessel with a reduced risk of causing vascular restenosis as compared to the use of multiple rigid stents which will make the narrow lumen narrower; and 2) their biodegradable part allows them to carry a therapeutic agent that is favorable to rapid tissue endothelialization and leads to reduced damage of them as a foreign object to the human body due to the self-degradation of this biodegradable part in the human body over time.

The forgoing description of the embodiments disclosed herein enables those skilled in the art to implement or use the present invention. Various modifications of these embodiments are obvious to those of ordinary skill in the art. The general principles as defined herein are applicable to other embodiments without departing from the spirit or scope of the present invention. Thus, the present invention is not limited to the disclosed embodiments, but rather it covers all those within the broadest scope consistent with the principles as defined herein.

## Claims

1. A luminal stent having a tubular mesh structure, wherein the tubular mesh structure is formed of metal filaments braided with biodegradable filaments.

2. The luminal stent according to claim 1, wherein the metal filaments are interlaced with the biodegradable filaments.

3. The luminal stent according to claim 2, wherein a ratio of a number of the metal filaments to a number of the biodegradable filaments is from 1:1 to 1:3.

4. The luminal stent according to claim 1, wherein the metal filaments are twisted together with the biodegradable filaments to form composite strands, and wherein the tubular mesh structure is braided from the composite strands.

5. The luminal stent according to claim 4, wherein a ratio of a number of the metal filaments to a number of the biodegradable filaments which are twisted together to form the composite strands is from 1:1 to 1:3.

6. The luminal stent according to any one of claims 1 to 5, wherein the metal filaments are flexible filaments of one or more metals selected from a group consisting of shape memory alloys, stainless steels and platinum-iridium alloys, the shape memory alloys being one or more selected from a group consisting of nickel-titanium-based shape-memory alloys, copper-based shape-memory alloys and iron-based shape-memory alloys.

7. The luminal stent according to any one of claims 1 to 5, wherein the biodegradable filaments are filaments of one or more selected from a group consisting of poly(p-dioxanone), polylactic acid and poly(glycolide-co-lactide).

8. The luminal stent according to claim 1, wherein the luminal stent is an uncovered single-layered stent.

9. The luminal stent according to claim 1, wherein the luminal stent carries a therapeutic agent.

10. The luminal stent according to claim 1, wherein the luminal stent is an endovascular stent for medical use.

11. A method of fabricating a luminal stent, the method comprising: forming a lattice by a braiding process; and shaping the lattice into a tubular mesh stent, wherein the braiding process comprises braiding metal filaments together with biodegradable filaments to form the lattice.

12. The method according to claim 11, wherein the braiding process comprises interlacing the metal filaments with the biodegradable filaments.

13. The method according to claim 11, wherein a ratio of a number of the metal filaments to a number of the biodegradable filaments is from 1:1 to 1:3.

14. The method according to claim 11, wherein the method further comprises twisting the metal filaments together with the biodegradable filaments to form composite strands prior to the braiding process, and wherein the braiding process comprises braiding the composite strands to form the lattice.

15. The method according to claim 14, wherein a ratio of a number of the metal filaments to a number of the biodegradable filaments which are twisted together to form the composite strands is from 1:1 to 1:3.
